# EUROPEAN PATENT APPLICATION

(11) **EP 4 749 281 A2**
(43) Date of publication of application: **27.05.2026**
(21) Application number: 26170330.0
(22) Date of filing: 23.03.2023
(51) Int. Cl.: G01N 33/53

(54) **DETECTOR**

(30) Priority: 16.04.2022 GB 202205632
(62) Divisional of application: 23715222.8
(71) Applicant: UK NIVD Ltd, Basildon Essex SS13 1BY (GB)
(72) Inventor: Trigueros, Sonia, Basildon SS13 1BY (GB)
(74) Representative: Cleveland Scott York

(57) **Abstract**

A composition comprising gold nanoparticles which are linked to polyclonal antibodies. The composition may be used to detect the presence of a target which binds to the polyclonal antibody in solution. The binding of the target to the polyclonal antibody triggers a self-assembly of the polyclonal antibody and the gold nanoparticles. This self-assembly of the polyclonal antibody and the gold nanoparticles triggers a colour change in the surface plasmon of the gold nanoparticles which indicates the presence of the target.

## Description

The invention relates to compositions for detecting targets such as biomarkers, detectors for detecting targets such as biomarkers and associated methods.

The risk of a global pandemic caused by an infectious agent has been high for the last decade. The COVID-19 pandemic and its waves of infection have shown the need for practical diagnostic tools. The low efficiency, high-cost, and extended test reaction time of existing diagnostic tests have demonstrated the need for a change in diagnostic devices, moving from expensive and slow laboratory-based techniques to accessible, reliable, and rapid diagnostic tools.

International health agencies are making intense efforts to manage the pandemic by exploring every aspect of therapy development, focusing on innovative diagnostic tools for rapid and selective detection of COVID-19 infection. Although the COVID-19 pandemic has brought to light the need for better diagnostic devices for infectious diseases, non-infectious diseases such as cancer and dementia are also in need of early diagnostic tools (and preferably tools which work in a pre-symptomatic or asymptomatic period), which will improve clinical decision making and medical treatment.

Better diagnostic tools are essential to deliver precision medicine, a medical model that proposes the customization of healthcare, with medical decisions, treatments, practices, or products being tailored to a subgroup of patients instead of a one-drug-fits-all model. In precision medicine, diagnostic testing can be employed for selecting appropriate and optimal therapies based on a patient's genetic make-up or other molecular or cellular analyses.

It is amongst the objects of the invention to provide products and methods for detecting targets such as biomarkers quickly or inexpensively or non-invasively or with a high sensitivity or a high specificity, and preferably with all of these characteristics. It is also amongst the objects of the invention to provide products and methods for an improved point of care diagnostic test for infectious diseases, non-infectious diseases or biomarkers more generally.

It is also amongst the objects of the invention to provide products and methods for detecting targets such as biomarkers for which the development process from the lab-scale to the pilot plant scale or the commercial scale is straightforward and/or low-cost.

It is also amongst the objects of the invention to provide products and methods for detecting targets such as biomarkers which do not require sophisticated laboratories or expert personnel, and/or are low-cost and/or easy to use.

It is also amongst the objects of the invention to provide products and methods for detecting targets such as biomarkers which overcome current limitations, such as lack of accuracy, lack of compatibility with different biological fluids, low temperature stability or lack of portability.

It is also amongst the objects of the invention to provide products and methods for detecting targets such as biomarkers which improve current diagnostic capacity concerning specificity, speed, or cost with enhanced sensitivity and/or reduced instrumentation size.

It is also amongst the objects of the invention to provide products and methods for detecting targets such as biomarkers which enable a reduction in sensor size. This is useful for providing portable, wearable, or implantable medical devices. It is also useful for the integration of biosensors with other medical instruments.

It is also amongst the objects of the invention to provide products and methods for detecting targets such as biomarkers at low concentrations and therefore potentially at an early stage of a condition. Early detection of biomarkers is particularly desirable for long term conditions such as cardiovascular diseases, cancer, chronic respiratory diseases and diabetes.

The inventions disclosed herein may improve the ability to detect infectious agents in their main reservoirs, such as animals and wastewater, and improve the global response plan for existing or potential health risk events.

In a first aspect the invention provides a composition according to claim 1.

Compositions according to the invention scatter light differently when the gold nanoparticles and polyclonal antibodies are dispersed in a liquid compared to when they are self-assembled in a liquid. Plasmons occur on the surface of gold nanoparticles. A plasmon is a coherent delocalized electron oscillation. Plasmon bands are the energy levels which are associated with a plasmon. The transition from dispersed gold nanoparticles to self-assembled gold nanoparticles causes shifts in the energies of the plasmon bands. Absorption by these varying energy bands provides visible colour transitions from red to blue and blue to red.

Compositions according to the invention may be dispersed in a liquid where they have a red colour. When surfaces of the nanoparticles are brought into contact, short-range interactions allow for nanoparticle to nanoparticle bonds and other interactions. As these bonds or interactions form, the solution changes colour from red to blue and sometimes to purple. These colour changes are plasmonically generated colour changes.

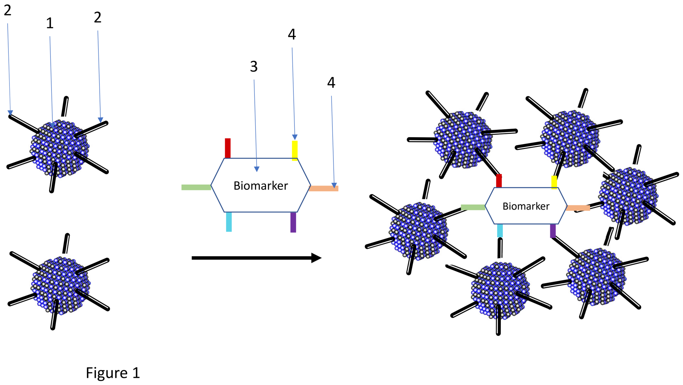

Compositions according to the present invention can use a reaction between a polyclonal antibody (linked to a gold nanoparticle) and a target (to which the polyclonal antibody binds) in a sample as the physical process which brings the nanoparticle surfaces into contact or interaction range. This results in a colour change from red to blue, which signals that the target, for example a biomarker, is present in a sample. Figure 1 shows a schematic representation of how compositions according to the invention undergo self-assembly in the presence of a biomarker. The gold nanoparticles 1 are linked, preferably covalently, to the polyclonal antibodies 2. The biomarker 3 from a sample which is added has several different regions 4 to which the antibodies 2 can bind. The binding of the polyclonal antibodies to different regions of the biomarker brings the nanoparticles close together (as shown on the right-hand side of the reaction) to provide the colour change from red (on the left of the reaction) to blue (on the right of the reaction).

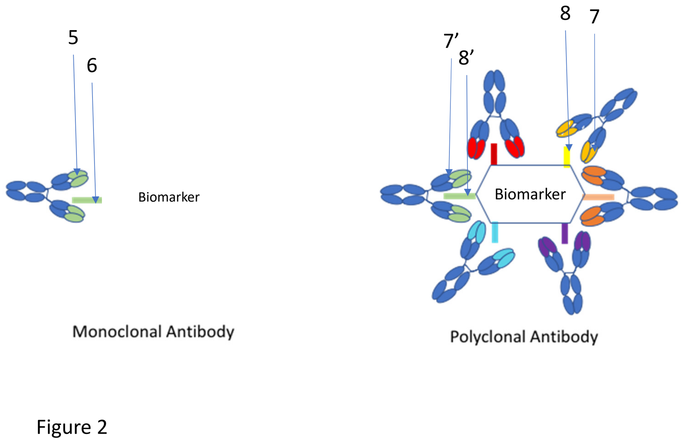

Figure 2 shows a schematic representation of how polyclonal antibodies bind to a biomarker in comparison with the binding of monoclonal antibodies to a biomarker. The monoclonal antibodies 5 only bind to one region 6 of the biomarker. This prevents them from providing the self-assembly effect which is provided by different parts 7 and 7' of the polyclonal antibodies binding to the corresponding (different) parts 8 and 8' of the biomarker.

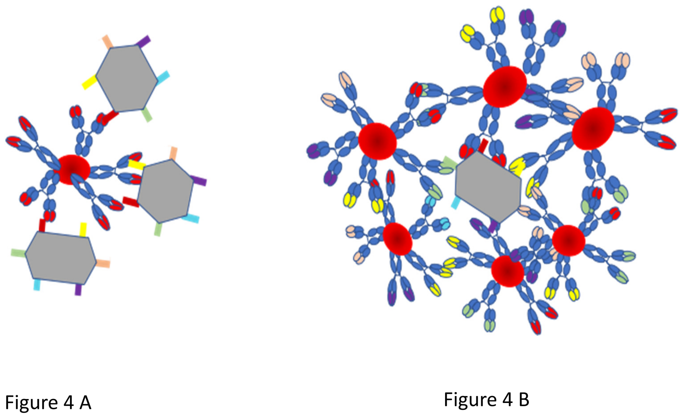

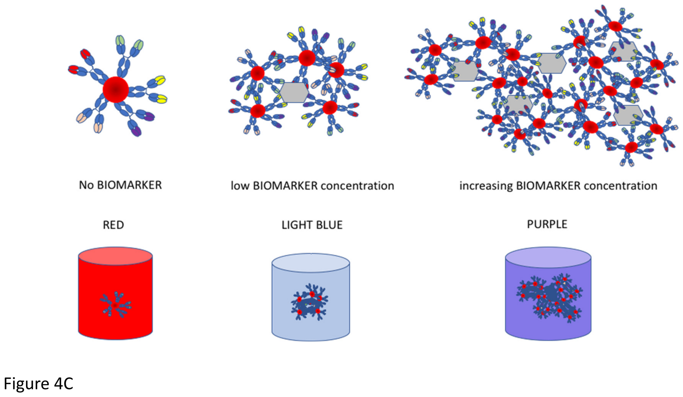

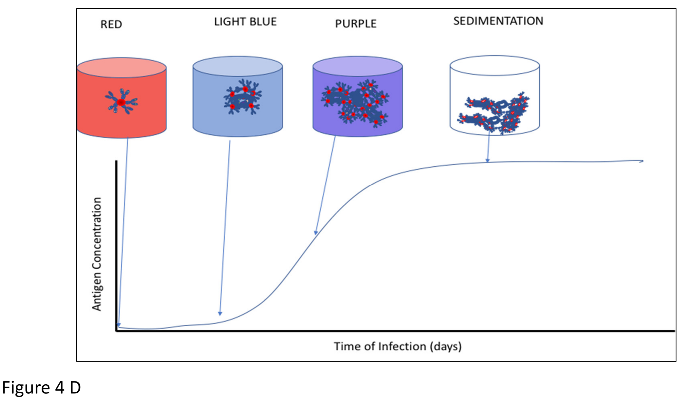

Figures 4A-4D show the binding of nanoparticles and monoclonal antibody complexes to a biomarker compared to the binding of nanoparticle and polyclonal antibody complexes to a biomarker. The monoclonal antibodies do not generate self-assembly (figure 4A) of the nanoparticles, whereas the polyclonal antibodies do generate self-assembly of the nanoparticles and a subsequent colour change from red to blue or purple. The colour change is shown in figure 4C. Biomarker concentration provides a colour change from red to blue. Figure 4D shows that the degree of colour change can provide an indication of the stage of infection in a patient. As the biomarker concentration increases with increasing infection, the colour change becomes darker until it reaches a grey colour. At very high concentrations of biomarker the effect may be washed out due to a quick and strong response consequently producing a sedimentation of the components.

The use of a polyclonal antibody linked to a gold nanoparticle can surprisingly provide a huge increase in the ability to detect ultra-low concentrations of targets which bind to the polyclonal antibody. The invention is therefore useful for detecting ultra-low concentrations of targets such as specific biomarkers. It is thought that the ability of polyclonal antibodies to self-assemble gold nanoparticles very effectively in response to a target molecule binding to the polyclonal antibody is due to the complex structure of the polyclonal antibody.

Polyclonal antibodies (pAbs) are secreted by different B cell lineages within the body (whereas monoclonal antibodies come from a single cell lineage). Polyclonal antibodies are immunoglobulin molecules that react against a specific target (which may be a biomarker), each identifying a different region (epitope) of the target. Therefore, a specific target will be detected by more than one antibody. Multiple interactions will occur between targets and polyclonal antibodies. This in turn draws the gold nanoparticles (to which they are attached) into a close, self-assembled structure, triggering the change in plasmon band energies and the resulting visible colour change.

Unpredictably, the invention can provide this technical effect with no natural aggregation of the 'nanoparticle and linked polyclonal antibody' complexes, which would otherwise hinder the colour transition. Natural aggregation in solution would hinder the sensitivity of the colour transition to a target by initiating a colour change without any target (such as a biomarker) being present.

The prefix 'nano' refers to the fact that at least one dimension of a material's structure is smaller than 100 nanometres (nm).

Compositions according to the invention and the associated methods may be easy to manufacture/carry out. The use of gold nanoparticles provides an acceptable and recognisable visual colour change. Gold nanoparticles are also non-toxic.

A biomarker is a measurable indicator of a biological state. By definition, a biomarker is "a characteristic that is objectively measured and evaluated as an indicator of normal biological processes, pathogenic processes or pharmacological responses to a therapeutic intervention". Generally, biomarkers can be physical biomarkers (for example proteins) and non-physical biomarkers (for example heart rate). The biomarkers referred to herein are physical biomarkers, for example those which can interact physically with a polyclonal antibody.

Furthermore, the process to scale-up from the lab-scale to the pilot plant-scale or commercial scale is straightforward and low cost.

Preferably, the polyclonal antibodies are each linked to the gold nanoparticles by a covalent bond.

Preferably, the polyclonal antibodies are each linked to the gold nanoparticles by a linker. The linker may comprise an N-hydroxysulfosuccinimide derived group. This provides a robust attachment of the polyclonal antibody to the nanoparticle.

The linker may comprise an ester linkage. This provides a robust attachment of the polyclonal antibody to the nanoparticle, which is straightforward to produce.

The polyclonal antibodies may each be attached to the gold nanoparticles via a primary amide group. This provides a robust attachment of the polyclonal antibody to the nanoparticle, which is straightforward to produce.

The polyclonal antibodies may be adapted to bind specifically to one of a peptide, a protein, a hormone, an antibody and an organic compound. The entities to which the polyclonal antibodies are adapted to bind are described herein as the targets.

Preferred targets and 'target and polyclonal antibody' combinations which are exemplified herein are as follows:
1. FLAG^{®} Peptide (DDDDK peptide) / Polyclonal Anti-DDDDK tag (binds to FLAG^{®} tag sequence) antibody (ab1162).
2. Recombinant Human Topoisomerase II alpha protein (ab159735)/ Polyclonal Anti-Topoisomerase II alpha antibody (ab12318).
3. Recombinant Anti-Human Topoisomerase II alpha antibody (ab52934) from Rabbit.
4. Polyclonal Goat Anti-Rabbit IgG H&L (ab182016).
5. Avian CORT / Anti-Corticosterone antibody produced in rabbit/used in birds (Sigma C8784 -100TST).
6. Human CORT Corticosterone CORT 500mg (Sigma 27840).
7. Noncommercial avian Stress Hormone CORT extraction and detection in Methanol.
8. Anti-Human IgG antibody (ab2410).
9. Anti-Human IgM antibody (ab26867).
10. Anti-Human IgA antibody (ab2411).

In preferred embodiments the polyclonal antibodies may be adapted to bind to one or more of amyloid beta peptide, CSF biomarkers, natriuretic peptide, IgG, IgM, IgA, CigG, ANA, anti-dsDNA (anti double strand DNA antibody), VOCs (volatile organic compounds such as hormones), CEA, Troponin T/C/I, CRP/CD27/CD38, CA/GP120/GP41, cortisol, adrenaline and norepinephrine. These biomarkers act as indicators for diseases selected from cancer, dementia, CVD, tuberculosis, malaria and HIV. Biomarkers for sepsis, cholera and pneumonia may also be detected by selecting the appropriate polyclonal antibody. Preferably the polyclonal antibody is adapted to bind with a specific biomarker associated with any of the medical conditions described herein, which include the following shown in Table 1:

**Table 1**

| TYPE of BIOMARKER | | Specific BIOMARMER |
|---|---|---|
| PEPTIDES | | |
| | Alzheimer's disease | Amyloid beta peptide |
| | Non AD- Neureodegeneration disorders | CSF biomarkers |
| | Heart Development and diseases | Natriuretic Peptide |

| ANTIBODIES | | |
|---|---|---|
| | General test for Infectious Diseses | IgG/IgM/igA |
| | Colorectal Cancer | CigG |
| | Viral Infectious Diseases | IgG/IgM/igA (specific) |
| | Autoimmune Diseases | ANA/anti-dsDNA |

| PROTEINS | | |
|---|---|---|
| | Cancer | VOCs /CEA/ |
| | Myocardial Infarction, Stroke | TroponinT,C,I |
| | TB | CRP/CD27/CD38 |
| | HIV | CA/GP120/GP41 |

| HORMONES | | |
|---|---|---|
| | Stress | Cortisol/ Adrenaline/Norepinephrine |

Table 1 shows examples of the classes of target which are detectable using the products and methods according to the invention. The examples in the detailed description below show data which indicates that a specific hormones, peptides, antibodies (both specific and non-specific) and proteins can be detected by the invention. Other targets falling under these class headings, including the specific biomarkers and other targets listed herein are also thought to be detectable by the invention, by virtue of their similar properties or structures and the general mechanism of action of polyclonal antibody binding.

Preferably the polyclonal antibody is adapted to bind with a virus such as Influenza A, B or HIV-1 CA protein, HIV-1 Envelope glycoprotein GP120HIV-1 GP120, GP 41, or components thereof such as spike proteins or nuclear proteins.

Preferably the polyclonal antibody is adapted to bind to an antigen or capsid protein, for example of viruses such as Respiratory Viral Infections (RVS). For example, the IgG Human anti-SARS-CoV-2 and any variants thereof may be linked to the nanoparticles. For example, Anti-SARS-CoV-2 (COVID-19) Spike antibody raised against recombinant SARS-CoV-2 (2019-nCoV) spike protein (S2) domain or Anti-SARS-CoV-2 (COVID-19) Nucleocapsid antibody raised against recombinant SARS-CoV-2 (2019-nCoV)) Nucleocapsid protein may be linked to the nanoparticles.

Preferably the polyclonal antibody is adapted to bind to biomarkers associated with Helicobacter pylori, Pox Virus, Tuberculosis (TB), Pneumonia or Vibrio cholerae.

The gold nanoparticles may be provided with a surface layer of polyethylene glycol (PEG). The layer may be a full or a partial coating. The layer is preferably applied before the gold nanoparticles are linked to the polyclonal antibodies. This increases the extent to which polyclonal antibodies can link to the nanoparticles. This provides a composition which is easier to manufacture and more sensitive to target molecules due to the greater number of polyclonal antibodies which are linked to the gold nanoparticle. The PEG molecules also provide a more stable nanoparticle and polyclonal antibody complex which is less likely to self-aggregate prematurely, or in the absence of a specific target.

Preferably the PEG molecules comprise a carboxylic acid group, which is preferably at, or adjacent to, the end of the PEG molecules. This enhances the effects stated above by providing a PEG molecule which more readily binds to polyclonal antibodies and forms a more robust bond, especially when using an NHS/EDC linking reaction.

Preferably, the polyethylene glycol chain length is between 1000-3000 and more preferably between 1000-2500.These ranges enhance the effect described above.

Preferably the gold nanoparticles are substantially spherical. The gold nanoparticles may be spherical.

The diameters of the gold nanoparticles may be greater than 3.5 nm. This provides an acceptable level of surface plasmon and therefore an acceptable level of visible colour to the composition. The diameters of the gold nanoparticles may be between 5-50 nm and preferably between 10-40 nm. These ranges provide an acceptable level of surface plasmon and therefore a strong visible colour to the composition, for easy identification of a colour change.

The composition may be a powder. The composition may be lyophilized. This provides a convenient form for transporting the composition which can be added to a liquid sample or to a different liquid, to which sample is added. The lyophilized or powdered forms of the composition are also useful for adding to detection devices which are shipped to users in a dry form. The powder or lyophilized form can be adhered to a surface of a detection device. The user then adds a sample or a separate liquid to carry out a detection test.

In a further aspect the invention provides a liquid comprising a composition as described herein, wherein the composition (which comprises the gold nanoparticles and polyclonal antibodies) is dispersed in the liquid. This provides a convenient medium into which a sample may be introduced for detection of a target in the sample.

The concentration of the composition which comprises the gold nanoparticles and polyclonal antibodies in the liquid may be such that the absorbance of the liquid is between 0.1 - 1 AU (absorbance units).

The liquid may comprise a liquid selected from the group of water, methanol, plasma, saliva, serum, sodium chloride solution having a concentration of between 50 mM and 200 mM, potassium chloride solution having a concentration between 50 mM and 200 mM, Magnesium Chloride solution (MgCl₂) having a concentration between 5 mM and 100 mM, PBS (Phosphate-buffered saline) having a concentration of below 10%, (preferably around 0.1%) and SBB (Sodium Borate Buffer) having a concentration of below 1M, (preferably around 0.1M). These liquids provide an acceptable low level of natural aggregation of the gold nanoparticles in the composition.

Ethanol is less preferred for use in the invention since it can lead to aggregation of the dispersed gold nanoparticle/polyclonal antibody complex without any target (to which the polyclonal antibody binds) being present. In preferred embodiments the solution contains substantially no ethanol.

In a further aspect the invention provides a method of manufacturing a composition as described herein comprising the step of linking gold nanoparticles to polyclonal antibodies.

The method may further comprise the step of attaching a linker to the gold nanoparticles prior to the linking of the gold nanoparticles to the polyclonal antibodies. This linker may be an activating group which increases the ability of the gold nanoparticles to link to polyclonal antibodies.

The step of attaching a linker to the gold nanoparticles may comprise the step of reacting the gold nano particles with N-(3-Dimethylaminopropyl)-N'-ethylcarbodiimide hydrochloride followed by N-hydroxysulfosuccinimide. This is known as an EDC, NHS reaction.

The step of linking the gold nanoparticles to the polyclonal antibodies may be conducted in the presence of polyethylene glycol (PEG). Equally, PEG may be applied to the nanoparticles before any polyclonal antibodies are linked to the nanoparticles. Preferably, the nanoparticles comprise a partial or full coating on their surface which comprises citrate groups, or other carboxylic acid derived groups. These groups create an activated partial or full coating which is well suited to further linking reactions. Preferably this activated partial or full coating is formed by treating uncoated gold nanoparticles with a sodium citrate or another carboxylic acid salt solution. Preferably the nanoparticles which comprise this activated partial or full coating are mixed with a liquid which comprises PEG so that a partial or full coating of PEG is provided over the activated partial or full coating.

The partial or full coating of PEG increases the extent to which polyclonal antibodies are linked to the nanoparticles. The number of polyclonal antibodies which may be attached to a PEG-treated nanoparticle is therefore greater than the number of polyclonal antibodies which may be attached to the corresponding non-treated nanoparticle. One mechanism by which the PEG treatment achieves this result is the increase in surface area which is provided by the PEG layer. The increased polyclonal antibody uptake by the PEG treated nanoparticles provides a composition which is easier to manufacture and more sensitive to target molecules due to the greater number of polyclonal antibodies which are linked to the gold nanoparticle. The partial or full coating of PEG on the nanoparticles is also thought to prevent unwanted natural aggregation (which could induce an unwanted colour change).

Preferably, the polyethylene glycol chain length is between 1000-3000 and preferably 1000-2500.This enhances the effect described above and ensures that the PEG treatment does not affect the plasmonic characteristics of the nanoparticles. The presence of the PEG partial or full coating improves the detection of small targets such as hormones and small (e.g. molecular) biomarkers.

Preferably before the gold nanoparticles are linked to the polyclonal antibodies (or the linker), the gold nanoparticles are treated such that they have citrate groups disposed on their surface, preferably in the form of a layer (as discussed above). This provides a functionalized surface to which a linker such as the product of an EDC-NHS reaction, or a polyclonal antibody can be easily and robustly linked.

In some embodiments a fluorescently labelled polyclonal antibody can be used to show that a bond between the gold nanoparticle and the polyclonal antibody has taken place. The fluorescence of the linked gold nanoparticle and polyclonal antibody complex may be detected using a fluorescence microscope.

In a further aspect the invention provides a method of detection of a target in a sample comprising the step of combining a composition as described herein or a liquid as described herein with a sample. The sample may be a liquid.

The sample may have a volume of between 3-5 microlitres. This is preferred because only a very small amount of composition containing the nanoparticles and polyclonal antibodies is required. This small volume is however still sufficient to give a recognisable result signal.

The liquid or sample may comprise a buffer having a pH in the range of 4-9, and preferably 4-8.5, 4-8 or 4-7. This prevents unwanted or premature aggregation of the nanoparticle and polyclonal antibody composition. A pH outside of these ranges is less preferred since it may drive aggregation by changing nanoparticles' surface charge.

The concentration of a target in the sample may be between 5 x 10⁻¹⁸ mole and 1 x 10⁻⁹ mole. This provides an ultra-low concentration detection method.

The sample may be a saliva sample, a urine sample, a soft tissue sample, a blood serum sample, a blood plasma sample, or an aqueous sample. This provides a wide range of flexibility for the sample being tested. In preferred embodiments the liquid and the sample will be of the same type. This can help to prevent unwanted or premature self-assembly. For example, if the sample to be run is a blood plasma sample, the liquid (containing the nanoparticle and polyclonal antibody complex, with which the sample is combined) may comprise a blood plasma.

The method may comprise a diagnostic step in which a colour change indicates the presence of a target which is a biomarker for a disease and thereby diagnoses the disease.

The combination of the composition or liquid and the sample may undergo a colour change from red to blue. This occurs when the sample contains a target to which the polyclonal antibody in the composition or liquid binds.

Preferably the solution undergoes a colour change in less than 3 minutes, and preferably less than 2 minutes and preferably less than 1 minute after the sample is combined with the solution, when the sample contains the target to which the polyclonal antibody binds.

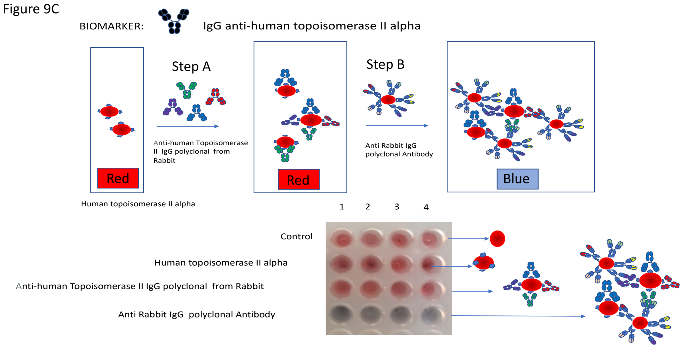

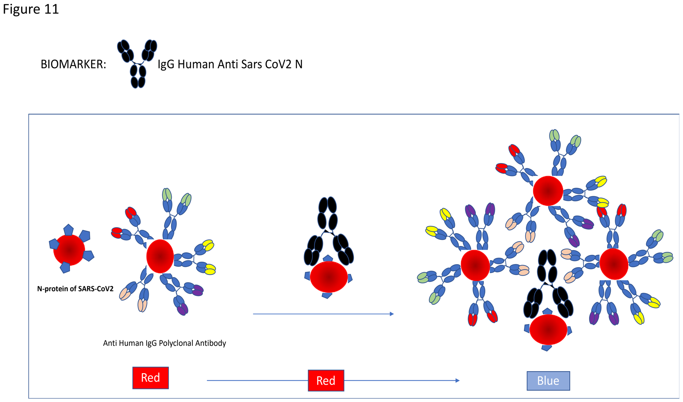

The target may be a biomarker and wherein, when the composition as described herein or the liquid as described herein is combined with the sample, gold nanoparticles each linked to a probe which binds to the biomarker are present. The benefits of this method are discussed and exemplified in figures 9C and 11 and the corresponding detailed description below. The method is useful for detecting specific types of antibodies which act as biomarkers for specific conditions.

The target may be an antibody which binds to the probe. The target may be a specific antibody which binds specifically to the probe. For example, the method may detect specific types of IgG or IgM or IgA antibodies.

The probe may be a protein such as a disease antigen or a part thereof, an enzyme or a peptide. The probe may be a spike protein or a Nucleocapsid protein associated with any of the conditions described herein.

In a further aspect the invention provides a detection test which comprises a first liquid or a first composition as described herein. The test may be a device.

The detection test may be adapted to carry out a method as described herein.

The detection test may be adapted to communicate a detection result to a remote location. It may do so via a wireless network and/or via the internet. The detection test may be connected to a mobile device such as a phone or tablet or computer in order to display the detection result or communicate the detection result to a remote location.

The detection test may comprise a second composition as described herein or a second liquid as described herein,
wherein the first composition as described herein or the first liquid as described herein and
the second composition as described herein or the second liquid as described herein
comprise different polyclonal antibodies such that the first and second compositions or liquids as described herein are adapted to detect different targets. That is, the test may comprise a combination of two different liquids, two different compositions or any combination of liquids and compositions, providing that the polyclonal antibody associated with each liquid/composition is different from the polyclonal antibody associated with the other liquid/composition.

The different targets may both be both biomarkers associated with a particular disease. For example, one biomarker may be the presence of a specific IgM antibody and the other biomarker (to which the relevant polyclonal antibodies bind) may be a specific IgG antibody. These embodiments can give a patient a fast and accurate way of assessing the stage of an infection by the presence or otherwise of the IgM and IgG antibodies. A hypothetical example of such a detector and method is provided in example 8 discussed below.

The first and second compositions or liquids may be spaced apart so that they may be reacted independently with different samples. This can allow a user to run, for example a blood sample, as well as a urine sample on the same detection test.

The first and second compositions or liquids may be adapted to be reacted simultaneously with a single sample or with different samples. This provides a fast test which is still able to detect multiple targets.

Embodiments of the invention will now be described with reference to the figures of the drawings and the following examples, in which;
Figure 1 shows a schematic representation of how compositions according to the invention undergo self-assembly in the presence of a biomarker.
Figure 2 shows a schematic representation of how polyclonal antibodies bind to a biomarker in comparison with the binding of monoclonal antibodies to a biomarker.
Figure 3 shows reaction steps which may be used to link a polyclonal antibody to a gold nanoparticle.
Figures 4A-4D show the binding of nanoparticles and monoclonal antibody complexes to a biomarker compared to the binding of nanoparticle and polyclonal antibody complexes to a biomarker, and the subsequent self-assembly and colour changes.
Figures 5A-5F show reaction steps which may be used to link a fluorescent polyclonal antibody to a gold nanoparticle and the fluorescence microscope images obtained in example 1.
Figures 6A-6D show the results of the detection of a protein/enzyme biomarker as described in example 2.
Figures 7A-7C show the results of the detection of a peptide biomarker as described in example 3.
Figures 8A-8D show the results of the detection of cortisone as described in example 4.
Figures 9A-9D show the results of detection of general and specific types of antibodies.
Figure 10 shows the reaction which occurs in the viral antigen sensor which is described in example 6.
Figure 11 shows the reaction which occurs in the viral serologic sensor which is described in example 7.
Figure 12 shows a graph of typical antibody production vs days since infection. An array also shows schematic results from when different targets are identified using a multi biomarker sensor according to the invention, as described in example 8.
Figure 13 shows arrays and schematic results from when different targets are identified using a multi biomarker sensor according to the invention, as described in example 9.

Example 1 describes a method of manufacture of a composition according to an embodiment of the invention.

Examples 2-5 describe exemplary compositions and associated targets which may be detected using methods or compositions according to the invention.

Examples 6-9 describe exemplary compositions and methods for detecting viruses and exemplary compositions, methods and devices for detecting multiple targets.

### Example 1

This example provides a method for covalently linking polyclonal antibodies to gold nanoparticles. The method demonstrates the benefit of a step in which a polyethylene glycol (PEG) full or partial coating is provided on the nanoparticles before the linking of the polyclonal antibody to the nanospheres. The general method used was as follows:
- Prepare Sodium Borate Buffer 1M (SBB) pH 8.5.
- Add, in a tube, 5 microliters of AuNPs (Optional addition of polyethylene glycol (PEG) to coat the nanoparticles before linking to the antibody).
- Add 10 microliters NHS (N-hydroxysulfosuccinimide) 0.2M.
- Add 5 microliters EDC (N-(3-Dimethylaminopropyl)-N'-ethylcarbodiimide hydrochloride) 0.2M. This step and the preceding step constitute the NHS-EDC linking reaction.
- Add 1 microliter of fluorescent polyclonal antibody at (2mg/ml).
- Add 494 microliters of 0.1M SSB solution. Keep overnight at room temperature (20 - 25°C) for linking reaction to take place
- Centrifuge at 10,000 rpm for 10 minutes, to separate the 'nanoparticle and polyclonal antibody' complex from the liquid.
- The AuNPs were then washed twice with 0.1 M (SBB) and resuspended in 50 µl of Sodium Borate Buffer 1M (SBB, pH 8.5).

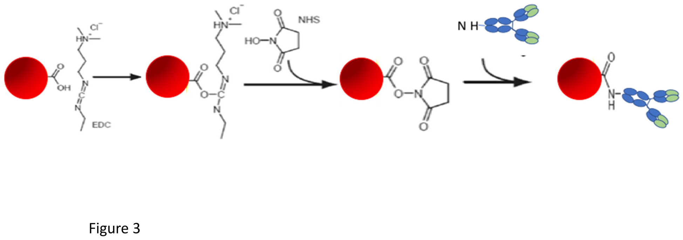

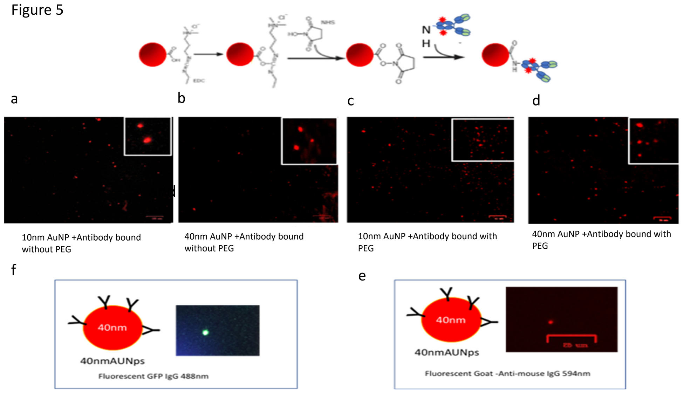

This method is shown schematically in figure 3 and at the top of Figure 5. The linking reaction is known as an EDC-NHS reaction. It provides a covalent bond between the polyclonal antibody and the gold nanoparticle surface.

10nm and 40nm diameter spherical AuNPs with and without the PEG treatment (see Figures 5A-5D) were covalently linked with Goat Anti-Mouse IgG H&L (Alexa Fluor^{®} 594), a fluorescent labeled antibody. An EDC-NHS reaction was used to link the antibody to the gold nanoparticles. The complex formed was analyzed by ZOE cell imager fluorescent microscope. The parameters used were as follows: RED Channel Excitation: 556/20 nm Emission: 615/61 nm.

Figures 5A-5D show the results. Figure 5A shows 10nm AuNP + Antibody bound without PEG. Figure 5B shows 40nm AuNP + Antibody bound without PEG. Figure 5C shows 10nm AuNP + Antibody bound with PEG. Figure 40nm AuNP + Antibody bound with PEG.

Figure 5F shows 40 nm AuNPs covalently linked with Goat Anti-Mouse IgG H&L (Alexa Fluor^{®} 488nm) fluorescent labeled antibody. The analysis is done by using a ZOE cell imager fluorescent microscope. The parameters used were as follows: GREEN Channel Excitation: 488 nm Emission: 515/45 nm.

The fluorescent marker here shows difference in efficiency of polyclonal antibody uptake between PEG-treated gold nanoparticles (figures 5C and 5D- high fluorescence) and nanoparticles which have not undergone the PEG treatment (figures 5a and 5b- low fluorescence).

### Example 2

In this example the target is a protein/enzyme biomarker. To test the invention reaction efficiency and specificity for protein/enzyme detection, the human anti-topoisomerase II antibody and human DNA topoisomerase 2-alpha protein were used. The invention proves to be accurate and sensitive when it scales down to the final volume needed. This invention is specific to the selected antigen.

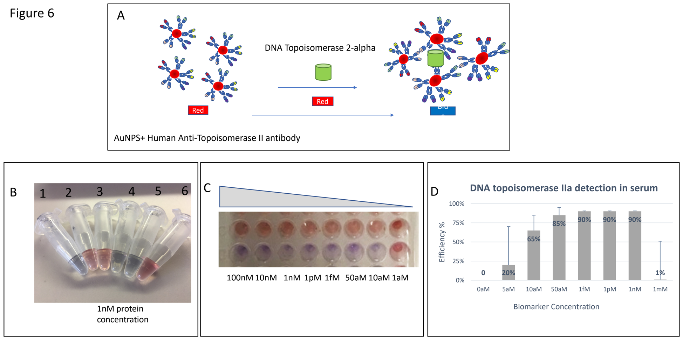

The results are shown in figure 6 in which;
A) Shows a schematic representation of protein detection.
B) Shows results from a reaction of in Eppendorf tubes with final volumes of 100 microliters:
   Sample tube 1 is the positive control. This tube shows the blue colour which has been induced (from a starting red liquid) by creating a pH change (to a high pH), or by adding large amount of salt.
   Sample tube 2 is 10 nanometer diameter AuNPs linked to monoclonal antibodies after addition of DNA topoisomerase 2-alpha protein. The colour is red.
   Sample tube 3 is 40 nanometer diameter AuNPs linked to monoclonal antibodies after addition of DNA topoisomerase 2-alpha protein. The colour is red.
   Sample tube 4 is 10 nanometer diameter AuNPs linked to polyclonal human anti-topoisomerase II antibody after addition of DNA topoisomerase 2-alpha protein. The colour is blue.
   Sample tube 5 is 40 nanometer diameter AuNPs linked to polyclonal human anti-topoisomerase II antibody after addition of DNA topoisomerase 2-alpha protein. The colour is blue.
   Sample tube 6 is the negative control, having only AuNPs linked to polyclonal human anti-topoisomerase II antibody. The colour is red.
   The reactions were done in serum as reaction buffer. In all cases a 1nM (nano mole) protein/biomarker concentration (DNA topoisomerase 2-alpha protein) was used.
   The monoclonal antibody sample tubes do not show a colour change from red to blue. The polyclonal antibody samples do show a colour change from a red starting solution to a blue solution on the addition of DNA topoisomerase 2-alpha protein.
C) Shows the plasmonic colour change using 40 nanometer diameter AuNPs and the Polyclonal antibodies mentioned above with decreasing biomarker concentrations. These decreasing concentrations are represented by the tapering wedge and the concentrations stated below the diagram. A final reaction volume of 3 microliters was used. The top row in this figure is a negative control, having only AuNPs linked to polyclonal human anti-topoisomerase II antibody. The colour is red in all cases. The second row shows varying concentrations of DNA topoisomerase 2-alpha protein which were added to the liquid containing the AuNPs linked to polyclonal human anti-topoisomerase II antibody. In the bottom row, all concentrations show a blue colour apart from the 1 aM (atto molar) concentration on the far right of the array, which remained red.
   Therefore, even at target concentrations as low as 10 x 10⁻¹⁸ i.e. 10 atto moles (10 aM) the polyclonal antibody nanoparticle complex provides a detectable colour change.
D) Shows reaction efficiency (%) as function of biomarker concentration. A positive 100% individual result was one in which the colour change was clear and took place in under 5 minutes. An individual result which did not attain these characteristics (e.g. unclear colour change or took longer than 5 minutes) was assigned a 0% reaction efficiency. The bars show an average efficiency taken over 25 individual tests done for each set of conditions. The results show that a good reaction efficiency is shown in biomarker concentrations even as low as 10 aM.

### Example 3

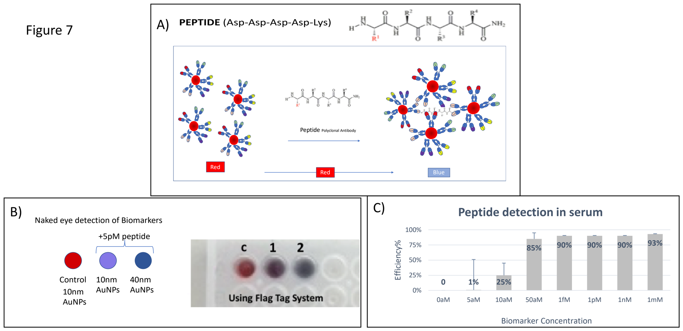

The target in this example is a peptide biomarker. The structure of the peptide and the results of this example are shown in figure 7. The peptide is a short chain of amino acids. Peptides are generally considered to be short chains of two or more amino acids. Due their small size, peptides are very difficult to detect. To test the reaction efficiency and specificity for peptide detection, the FLAG^{®} Expression System was used. We covalently linked the polyclonal antibody [Anti-DDDDK tag (which binds to FLAG^{®} tag sequence)(ab1162) that will specifically interact with FLAG^{®} Peptide (ddddk peptide) biomarker] onto 10 and 40 nanometre diameter gold nanoparticles.

The results are shown in figure 7A-7C.

Figure 7A shows a schematic representation of peptide detection and the associated colour change from red to blue.

Figure 7B shows the colour change indicating detection. On the right hand side is a photograph showing the colour change of 3 samples. On the left hand side is a schematic representation of the colour changes shown in the photograph.

Sample C in the photograph and the corresponding array in the schematic is the control sample with no peptide added. The liquid has a red colour.

Sample 1 (and the corresponding array in the schematic) shows the 10 nanometer diameter AuNP + Anti-DDDDK polyclonal antibody reaction in the presence of 5pM peptide. The liquid has a light purple colour.

Sample 2 (and the corresponding array in the schematic) shows the 40 nanometer diameter AuNP + Anti-DDDDK polyclonal antibody reaction in the presence of 5pM peptide. The liquid has a dark blue colour.

Figure 7C shows reaction efficiency (%) as function of biomarker concentration. Reaction efficiency was calculated as follows: A reaction efficiency of 100% for an individual result was assigned when the colour change was clear and took place in under 5 minutes. An individual result which did not attain these characteristics (i.e. unclear colour change or took longer than 5 minutes) was assigned a 0% reaction efficiency. The bars on the chart show an average reaction efficiency taken over 25 individual tests done for each set of conditions. The results show that peptide detection has an excellent efficiency even down to concentrations as low as 50 aM (atto molar).

### Example 4

It has been shown that parasite/viral/bacterial infections in animals produce an increase in stress hormone levels. To investigate this type of biomarker, the sensor was prepared to detect the presence of stress hormone corticosterone (CORT), isolated from avian samples. Commercial avian Anti-CORT antibody was covalently linked to 10 and 40 nm AuNPs. The stress hormone from feather samples was obtained by methanol extraction and quantified using a NanoDrop Spectrophotometer (A280). Low amounts of obtained CORT were added to a liquid according to the invention. The results show a colour change even after using an ultra-low amount of sample, demonstrating the efficiency of the invention. Efficiency is also shown in methanol samples, where no unspecific (i.e. off-target) aggregation of the 'nanoparticle + polyclonal antibody complexes' was detected. To prove the specificity, human commercial corticosterone (CORT) was also tested. If the test is specific, no colour change would be expected when the avian anti-CORT antibodies are used on human CORT samples. No colour change was detected using the human CORT. This shows the specificity of the invention (i.e. no cross-reaction with human versions of the biomarker - in this case a hormone).

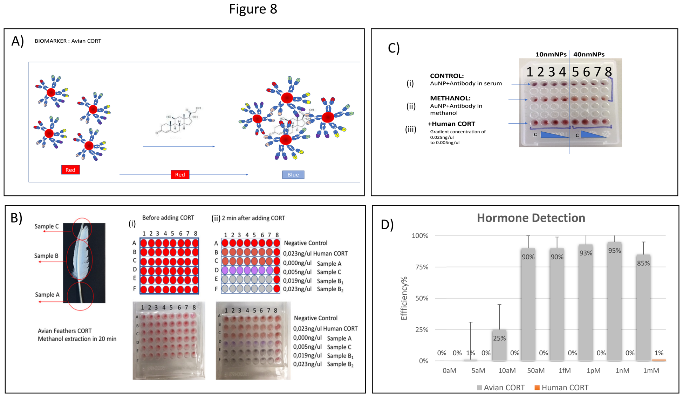

The results are shown in figure 8A-8D, in which;
Figure 8A shows a schematic representation of hormone detection reaction and the associated colour change from red to blue.

Figure 8B shows the results of tests using CORT hormone and the 'anti-avian CORT-antibody'. The schematic version of each array summarizes the colour changes shown in the photograph versions, for clarity. The CORT hormone was methanol extracted over 20 minutes from 3 parts of a feather (samples A, B and C). CORT concentrations obtained were measured by NanoDrop Spectrophotometer (A280). Array (i) shows the liquids according to the invention before adding any CORT. All of the liquids are red. Array (ii) shows the liquids after reaction with avian CORT and Human CORT.

Rows A (negative control), B (human CORT added) and row C (ultra-low concentration Avian CORT) show no colour change. Rows D-F corresponding to varying concentrations of avian CORT all show a colour change from red to blue, apart from column 8 which shows a negative control column.

Figure 8C shows an array to demonstrate reaction of gold nanoparticles linked to anti-avian CORT-antibody with Human CORT. Row i) shows a control comprising gold nanoparticles and antibody in serum. Row ii) shows a control comprising gold nanoparticles and antibody in methanol. Row iii) shows gold nanoparticles and antibody mixed with human cortisone. In the array, the columns 1-4 relate to 10 nm diameter nanoparticles. The columns 5-8 relate to 40 nm diameter nanoparticles. The wedges labelled C show the qualitative concentration of human cortisone in row iii). The concentrations range from 0.025 ng/microliter to 0.005ng/microliter. Both of the rows i) and ii) are red in colour as expected. Row iii) shows that no colour change (liquid remains red) is shown over varying concentrations of human cortisone, for both the 10 nanometre and 40 nanometre diameter nanoparticles. These results show that any colour change is only associated specifically with the detection of avian cortisone and not human cortisone, regardless of the concentration.

Figure 8D shows reaction efficiency (%) as function of biomarker concentration. Reaction efficiency was calculated as follows: A reaction efficiency of 100% for an individual result was assigned when the colour change was clear and took place in under 5 minutes. An individual result which did not attain these characteristics (i.e. unclear colour change or took longer than 5 minutes) was assigned a 0% reaction efficiency. The bars on the chart show an average reaction efficiency taken over 25 individual tests done for each set of conditions. The results demonstrate the excellent detection (90% reaction efficiency) of avian cortisone even down to concentrations as low as 50 aM (atto molar). They also demonstrate the specificity of the detection to avian cortisone over human cortisone. The human cortisone 'bars' are shown as either 0% or 1% for every cortisone concentration.

### Example 5

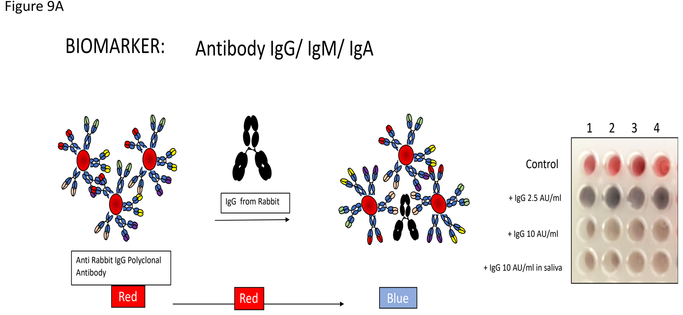

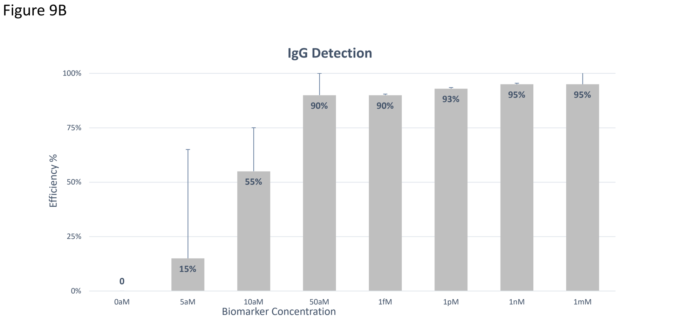

In this example the target is an antibody biomarker. In a first part to this example, anti-IgG, anti-IgM or anti-IgA polyclonal antibodies are linked to AuNPs and are used to detect the presence of IgG, IgM or IgA antibody biomarkers. The results are shown in figures 9A and 9B.

Figure 9A: Anti-Rabbit IgG polyclonal antibodies were covalently linked to 40 nanometre diameter AuNPs. 2.5 AU/ml (active units per ml) and 10 AU/ml samples of Rabbit IgG were used as the biomarker for detection in a reaction buffer. A 10 AU/ml Rabbit IgG sample in saliva was also tested. In all the samples, a colour change from red to blue is induced by the presence of the biomarkers IgG. The red top row is a negative control. Result rows 1-4 in the array show repeated samples.

Figure 9B: This figure shows reaction efficiency (%) as function of biomarker concentration. Reaction efficiency was calculated as follows: A reaction efficiency of 100% for an individual result was assigned when the colour change was clear and took place in under 5 minutes. An individual result which did not attain these characteristics (i.e. unclear colour change or took longer than 5 minutes) was assigned a 0% reaction efficiency. The bars on the chart show an average reaction efficiency taken over 25 individual tests done for each set of conditions. The results demonstrate an excellent reaction efficiency, even down to concentrations as low as 50 aM (atto molar).

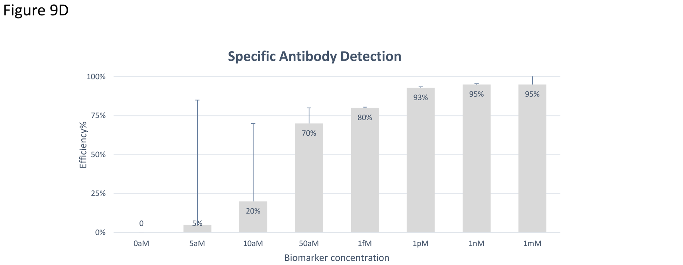

In the second part of this example, figures 9C and 9D show the detection of specific types of IgG or IgM or IgA antibodies. The antibody to be detected is the IgG anti-Human DNA topoisomerase II.

Figure 9C: Specific antibody detection. DNA topoisomerase II was covalently linked to 40 nanometre diameter AuNPs. This liquid is red. The Human DNA topoisomerase II alfa can be thought of as a probe which is linked to the gold nanoparticles to provide a starting material.

In step A, anti-human Topoisomerase II IgG polyclonal antibody from rabbit was added as a biomarker to the starting material (nanoparticles + probe). The liquid remains red. The antibody added binds specifically to the DNA topoisomerase II which is bound to the nanoparticle surface, without causing aggregation of the nanoparticles. The AuNPs with the Anti IgG polyclonal antibody now attached will now recognise the presence of IgG anti-human topoisomerase II alpha.

In step B anti-Rabbit IgG polyclonal Antibody was added into the reaction as a colour change inducer. The array shows the red top row (negative control). Columns 1-4 show repeated samples. The first, second and third rows are all red. Colour change from red to blue is only induced in the bottom row by the presence of the specific Anti-human DNA topoisomerase II IgG. The colour change will take place only in the presence of this specific IgG. In this example it was possible to detect a range of 1.5-10 AU/ml of the specific antibody in serum.

Figure 9D shows reaction efficiency (%) as function of biomarker concentration. Reaction efficiency was calculated as follows: A reaction efficiency of 100% for an individual result was assigned when the colour change was clear and took place in under 5 minutes. An individual result which did not attain these characteristics (i.e. unclear colour change or took longer than 5 minutes) was assigned a 0% reaction efficiency. The bars on the chart show an average reaction efficiency taken over 25 individual tests done for each set of conditions. The results demonstrate excellent reaction efficiency even down to concentrations as low as 50aM (atto-molar) or 1 fM (femto-molar).

The results shown in figures 9C and 9D show that a two-step technique can be used to detect specific antibodies (which themselves bind to specific targets associated with a specific disease).

In summary, a biomarker associated with a disease may be bound to the surface of a nanoparticle. A test sample which is thought to contain the corresponding specific antibody can be added to the liquid. The liquid remains red, whether or not the sample contains the target antibody. The presence of the specific antibody (if present) can then be detected by adding a polyclonal antibody (linked to gold nanoparticles) which binds only to the specific antibody to be detected. If the specific antibody is present a colour change will be induced from red to blue.

### Example 6

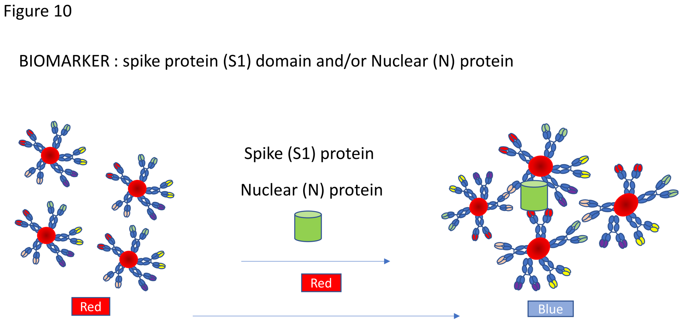

This example provides a viral antigen sensor, shown schematically in figure 10.

As an example of viral detection, an Anti-SARS-CoV-2 (COVID-19) Spike (S2) antibody raised against recombinant SARS-CoV-2 (2019-nCoV) spike protein (S2) domain or Anti-SARS-CoV-2 (COVID-19) Nucleocapsid protein (N) antibody raised against recombinant SARS-CoV-2 (2019-nCoV) Nucleocapsid protein (N), can be covalently linked to gold nanoparticles using the EDC-NHS reaction (see example 1).

The in vitro system can use a SARSCoV-2 Spike Glycoprotein (S2) and Nucleocapsid (N) protein which are both commercial recombinant antigens. The complexed form can be characterized prior to its use by Scanning Electron Microscopy - Energy Dispersive X-ray spectroscopy (SEM-EDX).

The detector can alternatively be designed to detect the presence of other viruses such as Influenza A, B or HIV-1 CA protein, HIV-1 Envelope glycoprotein GP120HIV-1 GP120, GP 41.

Antigen detection or capsid protein detection approach can be used to detect the presence of other viruses such as Viral Respiratory Infections (RVS). The IgG Human anti-SARS-CoV-2 may be linked to the gold nanoparticles.

### Example 7

This example provides a viral serologic-IgG/IgM detector. This is shown schematically in figure 11.

Two sets of nanoparticles are prepared by the EDC-NHS link reaction described herein. First, a set of nanoparticles is linked with the COVID antigen as previously described in example 6. The Covid antigen forms the probe. Second, a set of nanoparticles is linked with the Anti-human IgG or human IgM polyclonal antibodies. Both of these anti-human antibodies are commercial and do not require any lab preparation. The purpose of this sensor is to detect the specific human IgG or IgM in a sample which are generated as an immune response against the COVID-19 virus.

When the sample containing the IgG or IgM antibodies is added to the test liquid (which comprises the probe/antigen-linked nanoparticles and the 'anti-human IgG/IgM polyclonal antibody + nanoparticle' complex) a colour change is caused from red to blue. This is due to the IgG/IgM in the sample binding to the probe/antigen linked nanoparticle which bound complex is then associated with the polyclonal antibody/nanoparticle complexes to give the self-assembly.

This example is shown schematically in figure 11 which shows an IgG Human Anti-SARS-CoV-2 N protein detection system.

### Example 8

This example provides a multi-biomarker detector (Antigen/IgG/IgM/) in a single chip.

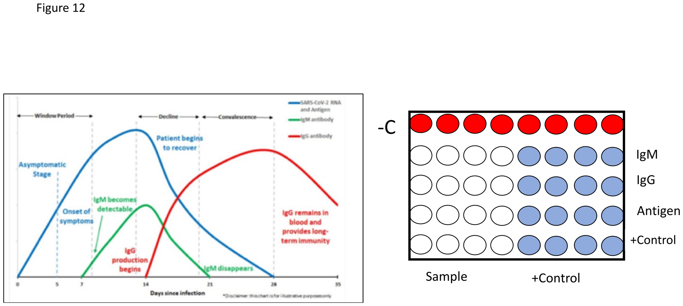

The design is shown in Figure 12. By providing a chip which comprises compositions according to the invention for detecting IgM as well as IgG antibodies, a user can be informed about the stage of disease. The graph in figure 12 shows the abundance of various antibodies vs time in a viral (covid infection). The earliest curve shows the increase and subsequent decrease in SARS-CoV-2 RNA and antigen present in the body. The second earliest curve shows the generation of IgM antibodies at an early stage of infection. As this curve declines, a third curve showing that the production of IgG antibodies takes the place of the IgM antibodies. Therefore, the presence of IgG antibodies, as detected by the test can show that the infection is late stage, whereas the detection of only IgM antibodies shows that the patient is still at an early stage of infection. This information can be coupled with the test for the presence of the specific disease antigen provided by the 4^{th} row of the array/device. This information is provided in a single test.

The array shown has a negative control top row which remains red. The rows 1-4 on the left are the clear coloured sample only. The rows 4-8 on the right show the blue colour induced when the red composition or liquid according to the invention is added to the samples which contain the biomarkers stated on the right-hand side of the array.

### Example 9

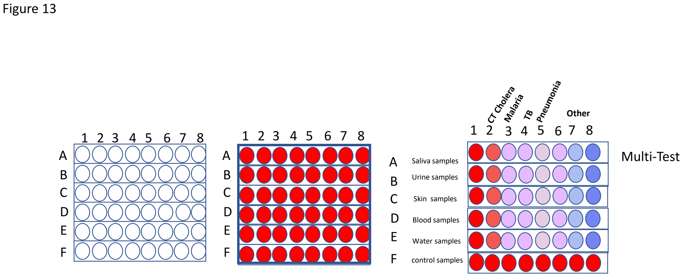

This example describes a multi pathogen detector in a single device. The device, which may be a chip, is shown in figure 13.

Due to the ability to adapt the invention to different biomarkers, methods and devices according to the invention can detect more than one type of biomarker from a single sample (e.g., a single blood, urine, saliva sample).

The methods and devices may also be able to test 10 or more samples. Suitable infectious agents for this methodology include Helicobacter pylori, Pox Virus, Tuberculosis (TB), Pneumonia and Vibrio cholerae. With this multiarray detector, one or more selected biomarkers specific to each infectious pathogen can be tested for. The same sensor can be adapted to non-infectious diseases like cancer and dementia, by selecting appropriate polyclonal antibodies to bind biomarkers or multiple biomarkers associated with those diseases. These embodiments of the invention are useful because more than one biomarker is often needed for proper diagnostic validation in cancer and dementia.

Figure 13 shows a schematic representation of a multi-biomarker nano-sensor detection in one chip. This embodiment can detect one or more biomarkers to detect different infectious diseases and from different samples simultaneously. This multi-biomarker nano-sensor could be used as a first-round broad-spectrum diagnostic tool for infectious agent identification.

The left-hand array shows the samples before addition of a liquid or composition according to the invention (all are clear coloured). The middle array shows the samples immediately upon addition of the composition or liquid according to the invention (all are red coloured). The array on the right shows the colour changes where the compositions/liquids according to the invention have reacted with the samples to provide blue or purple wells in the array. The negative control on the bottom row remains red.

Further statements of invention in numbered paragraphs are set out below.
Statement of invention 1: A composition comprising gold nanoparticles and polyclonal antibodies, wherein the polyclonal antibodies are linked to the gold nanoparticles.
Statement of invention 2: A composition according to statement of invention 1 wherein the polyclonal antibodies are each linked to the gold nanoparticles by a covalent bond.
Statement of invention 3: A composition according to either preceding statement of invention wherein the polyclonal antibodies are each linked to the gold nanoparticles by a linker.
Statement of invention 4: A composition according to statement of invention 3 wherein the linker comprises a N-hydroxysulfosuccinimide derived group.
Statement of invention 5: A composition according to either of statement of inventions 3 or 4 wherein the linker comprises an ester linkage.
Statement of invention 6: A composition according to any of statements of invention 3-5 wherein the polyclonal antibodies are each attached to the gold nanoparticles via a primary amide group.
Statement of invention 7: A composition according to any preceding statement of invention wherein the polyclonal antibodies are adapted to bind specifically to one of; a peptide, a protein, a hormone, and an antibody.
Statement of invention 8: A composition according to any preceding statement of invention wherein the polyclonal antibodies are adapted to bind to one or more of amyloid beta peptide, CSF biomarkers, natriuretic peptide, IgG, IgM, IgA, CigG, ANA, anti-dsDNA, VOCs, CEA, Troponin T/C/I, CRP/CD27/CD38, CA/GP120/GP41, cortisol, adrenaline and norepinephrine.
Statement of invention 9: A composition according to any preceding statement of invention wherein the gold nanoparticles are provided with a surface layer of polyethylene glycol.
Statement of invention 10: A composition according to statement of invention 9 wherein the polyethylene glycol chain length is between 1000-3000 and preferably 1000-2500.
Statement of invention 11: A composition according to any preceding statement of invention wherein the gold nanoparticles are substantially spherical. Statement of invention 12: A composition according to any preceding statement of invention wherein the diameters of the gold nanoparticles are greater than 3.5 nm.
Statement of invention 13: A composition according to any preceding statement of invention wherein the diameters of the gold nanoparticles are between 5-50 nm.
Statement of invention 14: A composition according to any preceding statement of invention wherein the composition is a powder.
Statement of invention 15: A composition according to any preceding statement of invention wherein the composition is lyophilized.
Statement of invention 16: A liquid comprising a composition according to any of statements of invention 1-15, wherein the composition which comprises the gold nanoparticles and polyclonal antibodies is dispersed in the liquid.
Statement of invention 17: A liquid according to statement of invention 16 wherein the concentration of the composition which comprises the gold nanoparticles and polyclonal antibodies is such that such that the absorbance of the liquid is between 0.1-1 AU.
Statement of invention 18: A liquid according to either of statements of invention 16 or 17 wherein the liquid comprises a liquid selected from the group of water, methanol, plasma, saliva, serum, sodium chloride solution having a concentration of between 50mM and 200mM, potassium chloride solution having a concentration between 50mM and 200mM, magnesium chloride solution having a concentration between 5mM and 100mM, 0.1M SSB, and PBS having a concentration of below 10%.
Statement of invention 19: A method of manufacturing a composition according to any of statements of invention 1-15 comprising the step of linking gold nanoparticles to polyclonal antibodies.
Statement of invention 20: A method according to statement of invention 19 comprising the step of attaching a linker to the gold nanoparticles prior to the linking of the gold nanoparticles to the polyclonal antibodies.
Statement of invention 21: A method according to statement of invention 20 wherein the step of attaching a linker to the gold nanoparticles comprises the step of reacting the gold nanoparticles with N-(3-Dimethylaminopropyl)-N'-ethylcarbodiimide hydrochloride followed by N-hydroxysulfosuccinimide.
Statement of invention 22: A method according to any of statements of invention 19-21, wherein the step of linking the gold nanoparticles to the polyclonal antibodies comprises a step in which polyethylene glycol is added to the gold nanoparticle before the gold nanoparticle is linked to the polyclonal antibodies.
Statement of invention 23: A method of detection of a target in a sample comprising the step of combining a composition according to any of statements of invention 1-15 or a liquid according to any of statements of invention 16-18 with a sample.
Statement of invention 24: A method according to statement of invention 23 wherein the sample has a volume of between 3-5 microlitres.
Statement of invention 25: A method according to either of statements of invention 23 or 24 wherein the liquid according to any of statements of invention 16-18 or the sample comprises a buffer having a pH in the range of 4-9.
Statement of invention 26: A method according to any of statements of invention 23-25 wherein the concentration of a target in the sample is between 5 x 10⁻¹⁸ mole and 1 x 10⁻⁹ mole.
Statement of invention 27: A method according to any of statement of inventions 23-26 wherein the sample is a blood plasma sample, a saliva sample, a urine sample, a soft tissue sample, a blood serum sample, or an aqueous sample.
Statement of invention 28: A method according to any of statements of invention 23-27 wherein the method comprises a diagnostic step in which a colour change indicates the presence of a target which is a biomarker for a disease and thereby diagnoses the disease.
Statement of invention 29: A method according to any of statements of invention 23-28 wherein the combination of the composition or liquid and the sample undergoes a colour change from red to blue.
Statement of invention 30: A method according to any of statements of invention 23-29 wherein the target is a biomarker and wherein, when the composition according to any of statements of invention 1-15 or the liquid according to any of statements of invention 16-18 is combined with the sample, gold nanoparticles each linked to a probe which binds to the biomarker are present.
Statement of invention 31: A method according to statement of invention 30 wherein the target is an antibody which binds to the probe.
Statement of invention 32: A method according to either of statements of invention 30 or 31 wherein the probe is a protein, an enzyme, a hormone, or a peptide.
Statement of invention 33: A detection test which comprises a first composition according to any of statements of invention 1-15 or a first liquid according to any of statements of invention 16-18.
Statement of invention 34: A detection test according to statement of invention 33 wherein the detection test is adapted to carry out the method of any of statements of invention 23-32.
Statement of invention 35: A detection test according to either of statements of invention 33 or 34 wherein the detection test is adapted to communicate a detection result to a remote location.
Statement of invention 36: A detection test according to any of statements of invention 33- 35 wherein the detection test comprises a second composition according to any of statements of invention 1-15 or a second liquid according to any of statements of invention 16-18, wherein the first composition according to any of statements of invention 1-15 or the first liquid according to any of statements of invention 16-18 and the second composition according to any of statements of invention 1-15 or the second liquid according to any of statements of invention 16-18 comprise different polyclonal antibodies such that the first and second compositions or liquids are adapted to detect different targets.
Statement of invention 37: A detection test according to statement of invention 36 wherein the different targets are both biomarkers associated with a particular disease.
Statement of invention 38: A detection test according to either of statements of invention 36 or 37 wherein the first and second compositions or liquids are spaced apart so that they may be reacted independently with different samples.
Statement of invention 39: A detection test according to any of statements of invention 36 to 38 wherein the first and second compositions or liquids are adapted to be reacted simultaneously with a single sample or with different samples.

## Claims

1. A composition comprising:
gold nanoparticles;
polyethylene glycol; and
polyclonal antibodies;
wherein the gold nanoparticles comprise a partial or full coating on their surface comprising carboxylic acid derived groups and the polyethylene glycol provides a partial or full coating over the partial or full coating of carboxylic acid derived groups; and
wherein the polyclonal antibodies are linked to the gold nanoparticles.

2. A composition according to claim 1 wherein the polyclonal antibodies are adapted to bind specifically to one of; a protein, a peptide, a hormone, and an antibody.

3. A composition according to either claim 1 or 2 wherein the polyclonal antibodies are adapted to bind to one or more of CRP/CD27/CD38, amyloid beta peptide, CSF biomarkers, natriuretic peptide, IgG, IgM, IgA, CigG, ANA, anti-dsDNA, VOCs, CEA, Troponin T/C/I, CA/GP120/GP41, cortisol, adrenaline and norepinephrine.

4. A composition according to any preceding claim wherein the polyethylene glycol chain length is between 1000-3000 and preferably 1000-2500.

5. A composition according to any preceding claim wherein the diameters of the gold nanoparticles are greater than 3.5 nm.

6. A liquid comprising a composition according to any of claims 1-5, wherein the composition which comprises the gold nanoparticles and polyclonal antibodies is dispersed in the liquid.

7. A liquid according to claim 6 wherein the liquid comprises a liquid selected from the group of water, methanol, plasma, saliva, serum, sodium chloride solution having a concentration of between 50mM and 200mM, potassium chloride solution having a concentration between 50mM and 200mM, magnesium chloride solution having a concentration between 5mM and 100mM, 0.1M SSB, and PBS having a concentration of below 10%.

8. A method of manufacturing a composition according to any of claims 1-5 comprising the step of linking gold nanoparticles to polyclonal antibodies.

9. A method according to claim 8, wherein the step of linking the gold nanoparticles to the polyclonal antibodies comprises a step in which polyethylene glycol is added to the gold nanoparticle before the gold nanoparticle is linked to the polyclonal antibodies.

10. A method of detection of a target in a sample comprising the step of combining a composition according to any of claims 1-5 or a liquid according to any of claims 6 or 7 with a sample.

11. A method according to claim 10 wherein the concentration of a target in the sample is between 5 x 10⁻¹⁸ mole and 1 x 10⁻⁹ mole.

12. A method according to either of claims 10 or 11 wherein the target is a biomarker and wherein, when the composition according to any of claims 1-5 or the liquid according to either of claims 6 or 7 is combined with the sample, gold nanoparticles each linked to a probe which binds to the biomarker are present.

13. A method according to claim 12 wherein the target is an antibody which binds to the probe.

14. A method according to either of claims 12 or 13 wherein the probe is a protein, an enzyme, a hormone, or a peptide.

15. A detection test comprising a first composition according to any of claims 1-5 or a first liquid according to either of claims 6 or 7 wherein the detection test comprises a second composition according to any of claims 1-5 or a second liquid according to either of claims 6 or 7,
wherein the first composition according to any of claims 1-5 or the first liquid according to either of claims 6 or 7 and the second composition according to any of claims 1-5 or the second liquid according to either of claims 6 or 7 comprise different polyclonal antibodies such that the first and second compositions or liquids are adapted to detect different targets.
